(19) Europäisches Patentamt
European Patent Office
Office européen des brevets

(11) Veröffentlichungsnummer : **0 026 894**
**B1**

(12) # EUROPÄISCHE PATENTSCHRIFT

(45) Veröffentlichungstag der Patentschrift : **30.03.83**

(21) Anmeldenummer : **80105871.0**

(22) Anmeldetag : **27.09.80**

(51) Int. Cl.$^3$ : **C 07 D401/06**// A61K31/47 ,(C07D401/06, 211/22, 215/14)

(54) **Verfahren zur Herstellung von erythro-alpha-2-Piperidyl-2,8-bis-(trifluormethyl)-4-chinolin-methanol.**

(30) Priorität : **05.10.79 DE 2940443**

(43) Veröffentlichungstag der Anmeldung : **15.04.81 Patentblatt 81/15**

(45) Bekanntmachung des Hinweises auf die Patenterteilung : **30.03.83 Patentblatt 83/13**

(84) Benannte Vertragsstaaten : **AT BE CH DE FR GB IT LI NL**

(56) Entgegenhaltungen :
**DE A 2 806 909**
**DE C 287 304**
**DE C 313 725**
**JOURNAL OF MEDICINAL CHEMISTRY, Band 14, Nr. 10, 1971, OHNMACHT, PATEL und LUTZ, « Antimalarials. 7. Bis(trifluoromethyl)-alpha-(2-piperidyl)-4-quinoline-methanols », Seiten 926-928**
**HELMUT KRAUCH und WERNER KUNZ « Reaktionen der organischen Chemie » 5. Auflage, 1976 DR. ALFRED HÜTHIG-VERLAG, Heidelberg, Seite 479**
**HELMUZ KRAUCH und WERNER KUNZ « Reaktionen der organischen Chemie » 5. Auflage, 1976 DR. ALFRED HÜTHIG-VERLAG, Heidelberg, Seiten 262, 263**

(73) Patentinhaber : **BASF Aktiengesellschaft**
**Carl-Bosch-Strasse 38**
**D-6700 Ludwigshafen (DE)**

(72) Erfinder : **Hickmann, Eckhard, Dr.**
**Comeniusstrasse 45**
**D-6700 Ludwigshafen (DE)**
Erfinder : **Oeser, Heinz-Guenter, Dr.**
**Friedrich-Burschell-Weg 19**
**D-6700 Ludwigshafen (DE)**
Erfinder : **Moebius, Leander, Dr.**
**Bahnhofstrasse 37**
**D-6701 Erpolzheim (DE)**

**EP 0 026 894 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von erythro-α-2-Piperidyl-2,8-bis-(trifluormethyl)-4-chinolin-methanol

Mefloquin, erythro-α-2-Piperidyl-2,8-bis-(trifluormethyl)-4-chinolinmethanol, eine Verbindung für die Behandlung auch der chloroquinresistenten Formen der Malaria, wurde bisher auf zwei Wegen, und zwar gemäß C. J. Ohnmacht et al., J. Med. Chem. *14,* 926-928 (1971) oder der DE-OS 28 06 909, hergestellt.

Nachteilig an den beiden Verfahren ist insbesondere das Arbeiten mit n-Butyllithium in Diethylether/n-Hexan-Gemischen bei − 70 °C. Diese Verfahren erfordern, wenn im technischen Maßstab gearbeitet wird, einen erheblichen sicherheitstechnischen Aufwand wegen der hohen Brandgefahr. Hinzu kommt der Aufwand wegen der unbedingt einzuhaltenden tiefen Temperaturen, da bei höheren Temperaturen vermehrt Nebenprodukte entstehen. Es bestand daher der Bedarf nach einem technisch leichter realisierbaren Herstellverfahren.

Die vorliegende Erfinding betrifft unter Vermeidung von lithiumorganischen Zwischenstufen ein vereinfachtes Verfahren zur Herstellung von erythro-α-2-Piperidyl-2,8-bis-(trifluormethyl)-4-chinolin-methanol, das dadurch gekennzeichnet ist, daß man 2,8-Bis-(trifluormethyl)-chinolin-4-carbonsäure oder deren Salz mit einem 2-Pyridylmagnesiumhalogenid zum 2-Pyridyl-2,8-bis-(trifluormethyl)-4-chinolyl-keton nach Art einer Grignard-Reaktion umsetzt und dieses in an sich bekannter Weise zum erythro-α-2-Piperidyl-2,8-bis-(trifluormethyl)-4-chinolin-methanol hydriert.

In einer bevorzugten Ausführungsform wird 2,8-Bis-(trifluormethyl)-chinolin-4-carbonsäure oder deren Salz verwendet, die hergestellt worden ist aus 7-Trifluormethyl-isatin durch Umsetzung mit 1,1,1-Trifluoraceton in Gegenwart einer starken Base, wobei das als Zwischenprodukt benötigte 7-Trifluormethylisatin zweckmäßigerweise durch Umsetzung von 2-Trifluormethyl-anilin mit Chloralhydrat und Hydroxylammoniumchlorid zum 2-Trifluormethyl-oximino-acetanilid und dessen Cyclisierung mit konz. Schwefelsäure in 10 bis 15 gew.%iger Lösung bei Temperaturen von 75 bis 85 °C und innerhalb von 0,5 bis 1,0 Stunden erhalten worden ist.

Diese Umsetzungen werden in den folgenden beiden Schemata veranschaulicht :

Schema 1

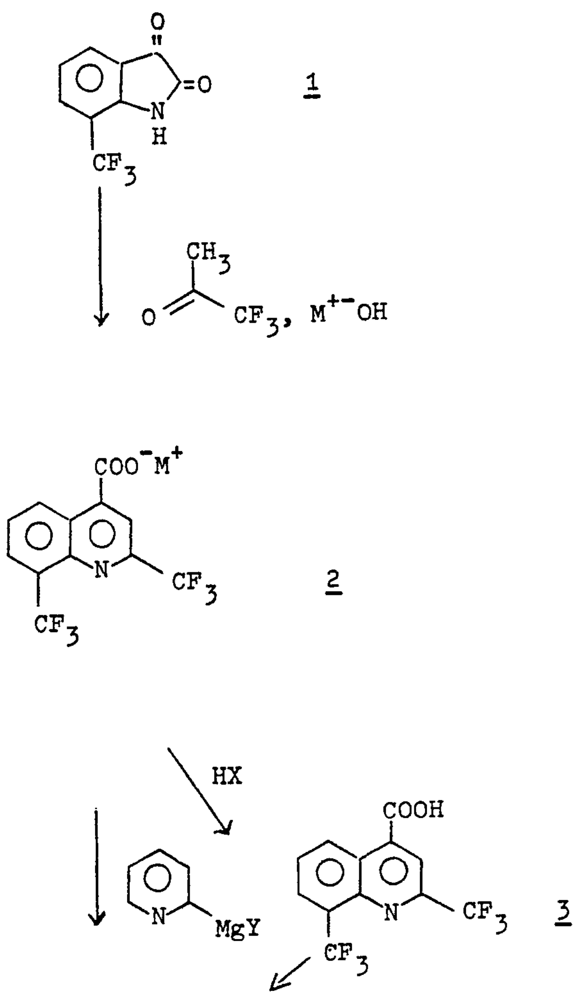


1

2

3

2

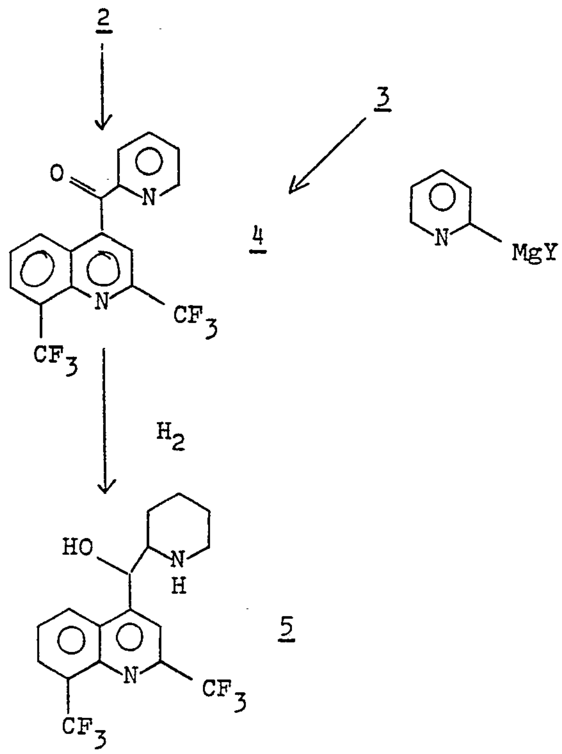


3

4

5

Dabei sollen M insbesondere ein Li-, Na- oder K-Atom, X Chlor und Y Chlor oder Brom bedeuten.

Schema 2

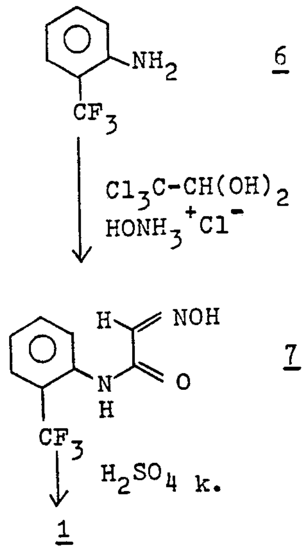


6

7

1

Die Umsetzung von 7-Trifluormethylisatin und 1,1,1-Trifluoraceton, in äquimolarer bis 100 Mol % überschüssiger Menge, erfolgt zweckmäßigerweise in Wasser als Lösungsmittel in Gegenwart von 0 bis 100 Mol % überschüssiger starker Base, bezogen auf das 7-Trifluormethyl-isatin und bei Temperaturen von 60 bis 150 °C unter Normaldruck oder gegebenenfalls in einem geschlossenen System.

Als starke Basen werden vorzugsweise die Alkalimetallhydroxide, Lithium-, Natrium- oder Kaliumhydroxid, verwendet, wobei das molare Mengenverhältnis 7-Trifluormethyl-isatin zu 1,1,1-Trifluoraceton zu Alkalimetallhydroxid 1 : 1-1,2 : 1-1,2 beträgt. Der bevorzugte Temperaturbereich liegt bei 60 bis 150 °C. Die Reaktion kann oberhalb von 100 °C in einem Druckgefäß durchgeführt werden.

Interessanterweise kann man vorteilhaft bei 80 bis ca. 100 °C Reaktionstemperatur unter Normaldruck arbeiten, obwohl die Siedetemperatur des eingesetzten 1,1,1-Trifluoracetons laut J. Amer. Chem. Soc. *69* 1819 (1947) bei 21 °C liegt. In der Regel ist die Reaktion innerhalb von 1 bis 20, bevorzugt 3 bis 10 Stunden beendet.

Die Aufarbeitung des Reaktionsgemisches erfolgt in an sich üblicher Weise durch Eindampfen des

Reaktionsgemisches, gegebenenfalls unter reduziertem Druck. Will man die freie Carbonsäure selbst darstellen, so säuert man das Reaktionsgemisch mit einer starken Säure, Beispielsweise mit wäßriger Salz- oder Schwefelsäure, auf pH 3 bis 1 an und isoliert den gebildeten Niederschlag. Falls gewünscht, kann aus der freien Carbonsäure ein Salz direkt hergestellt werden, das dann in besonders reiner Form anfällt.

Es wird darauf hingewiesen, daß eine etwa entsprechende Umsetzung für die Herstellung von in 2-Stellung substituierten Chinolin-4-carbonsäuren beispielsweise im Journal für praktische Chemie *56*, 283 bis 285 (1897) und in J. Amer. Chem. Soc. *53*, 318 ff. (1931) beschrieben wird. Nach diesem Stand der Technik ist ein sehr großer Alkaliüberschuß, das 4- bis 9-fache der berechneten Menge, erforderlich, und die Reaktion wird in homogener Lösung, beispielsweise durch Zusatz von Ethanol als Lösungsvermittler, durchgeführt, um optimale Ergebnisse zu erreichen. Überraschenderweise läßt sich die oben angegebene Reaktion bevorzugt bei nur 0 bis 20 Mol % überschüssigem Alkali und ohne Lösungsvermittler durchführen. Umsetzungen in Lösungsgemischen von Wasser mit Ethanol oder Tetrahydrofuran führen sogar zu unbefriedigenden Ausbeuten.

Die erhaltene 2,8-Bis-(trifluormethyl)-chinolin-4-carbonsäure oder eines ihrer Salze wird vorteilhaft in einem cyclischen gesättigten Ether oder einem aliphatischen Dialkylether oder einem Alkoxyalkylether mit einem 2-Pyridylmagnesiumhalogenid in einem Mengenverhältnis von 1 : 2 bis 1 : 8 Mol/Mol bei Temperaturen von 0 bis 80 °C umgesetzt.

Das bevorzugte 2-Pyridylmagnesiumhalogenid ist das Bromid. Als zweckmäßige Lösungs- oder Suspensionsmittel für die Carbonsäure oder ihre Salze kommen Tetrahydrofuran, Diethylether, Diisopropylesther, Methyl-tert.-butylether, Ethylenglykol-dimethylether oder Diethylenglykoldimethylether in Betracht. Diese Ether können weitere, unter den Reaktionsbedingungen inerte Cosolventien, wie beispielsweise Toluol oder Hexan, enthalten.

Die bevorzugten Salze für obige Reaktion sind die Alkalimetallsalze, insbesondere das Lithium-, Natrium- und Kaliumsalz, sowie das Magnesiumsalz.

Die bevorzugten Mengenverhältnisse liegen bei der Umsetzung der freien Carbonsäure bei 1 : 4 bis 1 : 8 Mol Carbonsäure pro Mol des zur Bildung der Grignardverbindung eingesetzten 2-Brompyridins, bei der Umsetzung eines Salzes der Carbonsäure bei 1 : 2 bis 1 : 4 Mol/Mol.

Die bevorzugten Temperaturen liegen bei 20 bis 60 °C und die Reaktion ist in der Regel innerhalb von 0,5 bis 5 Stunden beendet.

Es wurde gefunden, daß eine technisch ganz besonders vorteilhafte Verfahrensweise darin besteht, das 2-Pyridylmagnesiumhalogenid aus dem 2-Halogenpyridin und Magnesium in Gegenwart der 2,8-Bis-(trifluormethyl)-chinolin-4-carbonsäure oder ihrer Salze herzustellen und *in situ* mit der genannten Carbonsäure oder ihren Salzen zum 2-Pyridyl-2,8-bis-(trifluormethyl)-4-chinolyl-keton umzusetzen.

Die Isolierung des 2-Pyridyl-2,8-bis-(trifluormethyl)-4-chinolyl-ketons aus dem Reaktionsgemisch erfolgt beispielsweise durch Zersetzung der überschüssigen Grignardverbindung mit Wasser oder Mineralsäure, gegebenenfalls unter Kühlung, Abdampfen des Lösungsmittels und Extrahieren des Rückstandes mit einem organischen Lösungsmittel, beispielsweise Toluol oder n-Hexan. Vorteilhaft wird der Rückstand vor der Extraktion mit überschüssiger wäßriger Mineralsäure, beispielsweise Schwefelsäure oder Salzsäure, versetzt. Man kann auch den Eindampfrückstand nach der Zugabe von Mineralsäure mit einem Komplexierungsmittel für Magnesiumionen, beispielsweise Ethylendiamin-tetraessigsäure versetzen, dann mit einer wäßrigen Lauge, beispielsweise Natronlauge oder Kalilauge, alkalisch stellen, und mit einem organischen Lösungsmittel, beispielsweise Toluol oder n-Hexan, extrahieren. Ein recht reines 2-Pyridyl-2,8-bis-(trifluormethyl)-4-chinolyl-keton erhält man, wenn man ein durch das oben beschriebene Extrahieren mit Toluol erhaltenes Rohketon anschließend mit n-Hexan auskocht und das Keton aus der n-Hexanlösung durch Eindampfen und/oder Kristallisieren isoliert. Das erhaltene Keton kann weiter gereinigt werden durch Umkristallisieren, beispielsweise aus Methanol oder Ethanol, vorteilhaft in Gegenwart von Aktivkohle, oder durch Sublimation bei ca. 0,01 bis 0,1 mbar und ca. 100 °C Badtemperatur.

Überraschenderweise wurde gefunden, daß eine sehr vorteilhafte Aufarbeitungsmethode darin besteht, das Reaktionsgemisch direkt einzudampfen, den Eindampfrückstand der Grignardreaktion in einer höher konzentrierten Säure, insbesondere konzentrierter Salzsäure, wobei pro Teil Eindampfrückstand 1 bis 3 Teile wäßrige konzentrierte Salzsäure verwendet werden, zu lösen, die Lösung gegebenenfalls zu filtrieren und anschließend das 2-Pyridyl-2,8-bis-(trifluormethyl)-4-chinolyl-keton durch Verdünnen mit Wasser, wobei 1 bis 10 Teile Wasser pro Teil der verwendeten Säure verwendet werden, auszufällen.

Durch diese Verfahrensweise werden sonst schwer entfernbare Verunreinigungen, die im Dünnschichtchromatogramm (Merck-Fertigplatten Kieselgel 60 F 254 ; Laufmittel : Oberphase eines zuvor gut verrührten Gemisches aus 5 Volumenteilen Toluol, 5 Volumenteilen Eisessig und einem Volumenteil Wasser) an den in Nähe des Startpunktes verbleibenden, mit Joddampf intensiv braun anfärbenden Flecken zu erkennen sind, praktisch restlos entfernt.

Das beschriebene Reinigungsverfahren kann vorteilhaft auch zur Gewinnung des reinen, obengenannten Ketons aus stark verunreinigten Rückständen genutzt werden.

Die Hydrierung des Ketons zum erythro-α-2-Piperidyl-2,8-bis-(trifluormethyl)-4-chinolinmethanol erfolgt in an sich bekannter Weise, wie es beispielsweise in den beiden oben erwähnten Literaturstellen

beschrieben wird, z. B. an Platinkatalysatoren in alkoholischer Salzsäure. Dabei fällt das Mefloquin in Form seines Hydrochlorids an.

Das für das erfindungsgemäße Verfahren erforderliche 7-Trifluromethylisatin kann besonders vorteilhaft hergestellt werden, indem man o-Trifluormethylanilin mit Chloralhydrat und Hydroxylammoniumchlorid in einem molaren Verhältnis von 1 : 1,45 : 4,1 wäßriger, salzsaurer Natriumsulfathaltiger Lösung mit einem Gehalt von 2,5 bis 3,5 Gew.% o-Trifluormethylanilin, 0,6 bis 0,8 Gew.% Chlorwasserstoff und 17 bis 25 Gew.% Natriumsulfat. 10 $H_2O$ bei Temperaturen von 50 bis 100 °C, vorzugsweise bei 70 bis 90 °C umsetzt, und das direkt aus dem Reaktionsgemisch kristallin erhaltene 2-Trifluormethyloximinoacetanilid in konzentrierter Schwefelsäure in 10 bis 15 gew.%iger Lösung bei Temperaturen von 75 bis 85 °C innerhalb 0,5 bis 1 Stunde cyclisiert.

Diese Umsetzungen sind grundsätzlich bekannt, beispielsweise aus J. Amer. Chem. Soc. *73,* 3579-80 (1951) und analog der Vorschrift für die Herstellung von Isatin nach Org. Synth. Coll. Vol. I, 327-330 (1941). Jedoch unter den speziellen Bedingungen, insbesondere daß man einen Überschuß an Chloralhydrat verwendet, die Reaktionstemperatur auf 80 °C absenkt und in verdünnterer Lösung mit einer geringeren Konzentration an Natriumsulfat arbeitet, wird eine Ausbeuteverbesserung von 64 auf rund 80 % erreicht. Neben der Ausbeuteverbesserung, dem Arbeiten bei niedrigeren Temperaturen und mit geringeren Natriumsulfatmengen entfällt so zusätzlich eine Extraktion mit einem organischen Lösungsmittel. Das gleiche gilt für die Cyclisierungsreaktion zum 7-Trifluormethyl-isatin, wobei die gezielten Maßnahmen insbesondere darin bestehen, daß man in verdünnter Lösung während einer längeren Reaktionszeit bei erhöhter Temperatur cyclisiert und so zu einer erheblich verbesserten Ausbeute von fast 80 % gegenüber 60 % als Stand der Technik führen. Diese für eine Herstellung im technischen Maßstabe besonders hervorzuhebenden Vorteile sind umso bemerkenswerter, als in einem Temperaturbereich gearbeitet wird, der nach Literaturangaben (JACS *73,* 3579 und J. Org. Syn. Coll. Vol. I, 329) zu erheblichen Ausbeuteverlusten führen sollte.

In den Beispielen verhalten sich Teile zu Volumenteile wie Kilogramm zu Litern.

Beispiel 1

2,8-Bis-(trifluormethyl)-chinolin-4-carbonsäure

In einem Reaktionsgefäß mit Rührer, Kühler, Innenthermometer und Zudosiereinrichtung erhitzt man das Gemisch aus 100 Volumenteilen Wasser, 9,6 Teilen Natriumhydroxid, 43 Teilen 7-Trifluormethylisatin und 26,8 Teilen 1,1,1-Trifluoraceton unter Normaldruck 6 Stunden zum Sieden. Im Dünnschichtchromatogramm einer nach dieser Reaktionsdauer entnommenen, sauer aufgearbeiten Probe ist kein 7-Trifluormethyl-isatin mehr erkennbar. Man läßt daraufhin den Ansatz abkühlen und versetzt ihn unter Rühren mit 2 n Salzsäure, bis pH 2,5 erreicht ist. Das ausgefallene Produkt wird mit Wasser gewaschen und getrocknet. Man erhält 57 Teile 2,8-Bis-(trifluormethyl)-chinolin-4-carbonsäure, das sind 92,2 % der Theorie.

Beispiel 2

2,8-Bis-(trifluormethyl)-chinolin-4-carbonsäure

Man verfährt wie unter Beispiel 1 beschrieben, erwärmt jedoch das Reaktionsgemisch 25 Stunden auf 70 °C. Nach der gleichen Aufarbeitung erhält man 52 Teile 2,8-Bis-(trifluormethyl)-chinolin-4-carbonsäure, das sind 84,1 % der Theorie.

Beispiel 3

2,8-Bis-(trifluormethyl)-chinolin-4-carbonsäure

Das Gemisch aus 100 Volumenteilen Wasser, 5,01 Teilen Lithiumhydroxid, 43 Teilen 7-Trifluormethylisatin und 23,5 Teilen 1,1,1-Trifluoraceton wird 5 Stunden wie unter Beispiel 1 beschrieben erhitzt. Nach der gleichen Aufarbeitung erhält man 55 Teile 2,8-Bis-(trifluormethyl)-chinolin-4-carbonsäure, das entspricht 89,0 % der Theorie.

Beispiel 4

2,8-Bis-(trifluormethyl)-chinolin-4-carbonsäure

In einem Metallautoklaven mit Rührer und Innenthermometer wird das Gemisch aus 100 Volumenteilen Wasser, 5,7 Teilen Lithiumhydroxid, 43 g 7-Trifluormethyl-isatin und 26,8 g 1,1,1-Trifluoraceton unter Rühren 5 Stunden auf 110 °C erhitzt. Nach der unter Beispiel 1 beschriebenen Aufarbeitung erhält man 56 Teile 2,8-Bis-(trifluormethyl)-chinolin-4-carbonsäure, das sind 90,6 % der Theorie.

Beispiel 5

2,8-Bis-(trifluormethyl)-chinolin-4-carbonsäure

Man verfährt wie unter Beispiel 4 beschrieben, jedoch setzt man 12,2 Teile Kaliumhydroxid anstelle von Lithiumhydroxid ein und erhitzt das Gemisch 15 Stunden auf 90 °C. Nach der gleichen Aufarbeitung erhält man 58 Teile 2,8-Bis-(trifluormethyl)-chinolin-4-carbonsäure, das sind 93,8 % der Theorie.

Vergleichsbeispiel zu Beispiel 5

2,8-Bis-(trifluormethyl)-chinolin-4-carbonsäure

Man verfährt wie unter Beispiel 5 beschrieben, jedoch verwendet man anstelle des reinen Wassers das Gemisch aus 50 Volumenteilen Wasser und 50 Volumenteilen Ethanol. Nach der Aufarbeitung erhält man 43 Teile 2,8-Bis-(trifluormethyl)-chinolin-4-carbonsäure, das sind 69,6 % der Theorie.

Beispiel 6

2,8-Bis-(trifluormethyl)-chinolin-4-carbonsäure

Das Gemisch aus 100 Volumenteilen Wasser, 8,8 Teilen Natriumhydroxid, 43,2 Teilen 7-Trifluormethyl-isatin und 27,4 Teilen 1,1,1-Trifluoraceton wird im Autoklaven 8 Stunden auf 110 °C erhitzt. Nach dem Abkühlen setzt man unter Rühren so lange 2 n Salzsäure zu, bis der pH 2,7 erreicht ist, saugt den ausgefallenen Niederschlag ab und trocknet ihn bei 70 °C und ca. 20 mbar. Man erhält 60 Teile 2,8-Bis-(trifluormethyl)-chinolin-4-carbonsäure, das sind 97,1 % der Theorie.

Beispiel 7

2-Pyridyl-2,8-bis-(trifluormethyl)-4-chinolyl-keton

Unter Argon werden 14,6 Teile Magnesiumspäne und 200 Volumenteile Tetrahydrofuran vorgelegt. Man erwärmt auf 50 bis 58 °C, gibt ca. 0,05 Teile Jod und ca. 1 Volumenteil Tetrachlormethan zu und rührt, bis die braune Farbe des Jods nahezu verschwunden ist. Anschließend gibt man die Lösung von 32 Teilen 2-Brompyridin in 30 Volumenteilen Tetrahydrofuran bei einer Temperatur von 50 bis 60 °C zu und rührt nach beendeter Zugabe noch 30 Minuten bei 50 °C nach. Man dekantiert die dunkel gewordene Lösung vom ungelöst gebliebenen Magnesium ab und gibt sie unter Argon innerhalb von ca. 10 Minuten zur gerührten Suspension von 20 Teilen Lithiumsalz der 2,8-Bis-(trifluormethyl)-chinolin-4-carbonsäure in 100 Volumenteilen Tetrahydrofuran ; dabei steigt die Temperatur des Reaktionsgemisches von 24 auf 40 °C. Nach 30-minütigem Rühren ist dünnschichtchromatographisch in einer sauer aufgearbeiten Probe die 2,8-Bis-(trifluormethyl)-chinolin-4-carbonsäure nicht mehr nachweisbar. Daraufhin destilliert man das Lösungsmittel ab, nimmt den Rückstand in einem Gemisch aus 500 Volumenteilen Wasser und 150 Volumenteilen 2 n Salzsäure auf, versetzt die Lösung mit 148 Teilen Ethylendiamintetraessigsäure, z. B. als Titriplex III®, stellt durch Zugabe von konz. Natronlauge einen pH von ca. 12 ein, extrahiert diese Lösung kontinuierlich ca. 3 Stunden mit 2 000 Volumenteilen Toluol in einem Perforator und dampft den Toluolextrakt ein. Man erhält 21 Teile rohes 2-Pyridyl-2,8-bis-(trifluormethyl)-4-chinolyl-keton, das entspricht 89,4 % der Theorie. Durch zweimaliges Auskochen des rohen Ketons mit je 500 Volumenteilen heißem n-Hexan kann man 15,6 Teile reines 2-Pyridyl-2,8-bis-(trifluormethyl)-4-chinolylketon isolieren, das entspricht 66,4 % der Theorie.

Beispiel 8

2-Pyridyl-2,8-bis-(trifluormethyl)-4-chinolyl-keton

Man verfährt, wie unter Beispiel 7 beschrieben, gibt jedoch nach dem Starten der Grignardreaktion die Lösung des 2-Brompyridins in Tetrahydrofuran bei einer Temperatur von 30 bis 35 °C zu, daß die Reaktion nicht abbricht und setzt die erhaltene Grignardlösung mit der Suspension von 22,05 Teilen Kaliumsalz der 2,8-Bis-(trifluormethyl)-chinolin-4-carbonsäure in 70 Volumenteilen 1,2-Dimethoxyethan um. Nach der gleichen Aufarbeitung erhält man 20 Teile rohes 2-Pyridyl-2,8-bis-(trifluormethyl)-4-chinolyl-keton, das sind 85,1 % der Theorie. Durch die Behandlung mit n-Hexan erhält man 14,8 Teile reines Keton, das sind 63,0 % der Theorie.

Beispiel 9

2-Pyridyl-2,8-bis-(trifluormethyl)-4-chinolyl-keton

Man verfährt wie unter Beispiel 7 beschrieben, löst jedoch den nach beendeter Reaktion durch Abdestillieren des Lösungsmittels erhaltenen Rückstand (81 Teile) in 200 Teilen konzentrierter Salzsäure auf, filtriert von den unlöslichen Anteilen ab, fällt das Produkt durch Einrühren von 700 Teilen Wasser aus, filtriert es ab und trocknet es. Man erhält 19,3 Teile reines 2-Pyridyl-2,8-bis-(trifluormethyl)-4-chinolyl-keton, das sind 82,1 % der Theorie.

Beispiel 10

2-Pyridyl-2,8-bis-(trifluormethyl)-4-chinolyl-keton

Man verfährt wie unter Beispiel 7 beschrieben, setzt jedoch anstelle des Lithiumsalzes 9,45 Teile der freien 2,8-Bis-(trifluormethyl)-chinolin-4-carbonsäure in die Reaktion ein. Nach der Aufarbeitung erhält man 8,1 Teile rohes 2-Pyridyl-2,8-bis-(trifluormethyl)-chinolyl-keton, das sind 73,0 % der Theorie. Nach dem Behandeln mit n-Hexan erhält man 5,9 Teile reines Keton, das sind 52,1 % der Theorie.

Beispiel 11

2-Pyridyl-2,8-bis-(trifluormethyl)-4-chinolyl-keton

Man verfährt wie unter Beispiel 7 beschrieben, jedoch legt man 20 Teile Lithiumsalz der 2,8-Bis-(trifluormethyl)-chinolin-4-carbonsäure in 100 Volumenteilen Diethylether anstelle von Tetrahydrofuran vor. Man erhält 23 Teile rohes 2-Pyridyl-2,8-bis-(trifluormethyl)-4-chinolyl-keton, das entspricht 97,9 % der Theorie. Durch Auskochen mit n-Hexan gewinnt man daraus 17,3 Teile reines Keton, das entspricht 73,6 % der Theorie.

Beispiel 12

2-Pyridyl-2,8-bis-(trifluormethyl)-4-chinolyl-keton

Man führt die Reaktion wie unter Beispiel 11 beschrieben durch und arbeitet den nach dem Eindampfen des Reaktionsgemisches erhaltenen Rückstand wie unter Beispiel 9 beschrieben auf. Man erhält 18,1 Teile reines 2-Pyridyl-2,8-bis-(trifluormethyl)-4-chinolyl-keton, das sind 81,5 % der Theorie.

Beispiel 13

2-Pyridyl-2,8-bis-(trifluormethyl)-4-chinolyl-keton

24 Teile 2,8-Bis-(trifluormethyl)-chinolin-4-carbonsäure werden in 100 Volumenteilen Wasser mit 3, 2 Teilen Natriumhydroxid versetzt. Nach dem Eindampfen und Trocknen erhält man das Natriumsalz der 2,8-Bis-(trifluormethyl)-chinolin-4-carbonsäure in quantitativer Ausbeute.

10,9 Teile Magnesium und 24 Teile 2-Brompyridin werden in 170 Volumenteilen Tetrahydrofuran analog Beispiel 7 zum 2-Pyridylmagnesiumbromid umgesetzt, das in analoger Weise mit 16 Teilen des oben beschriebenen Natriumsalzes, suspendiert in 80 Volumenteilen Diethylether zur Reaktion gebracht wird. Nach Aufarbeitung erhält man 12 Teile rohes 2-Pyridyl-2,8-bis-(trifluormethyl)-4-chinolyl-keton, das sind 76,1 % der Theorie.

Vergleichsbeispiel zu Beispiel 13

2-Pyridyl-2,8-bis-(trifluormethyl)-4-chinolyl-keton

Man verfährt wie in Beispiel 6 beschrieben, arbeitet das Reaktionsgemisch jedoch durch Eindampfen auf das Natriumsalz der 2,8-Bis-(trifluormethyl)-4-chinolin-carbonsäure auf. Das rohe Natriumsalz (58 Teile), das noch im Überschuß eingesetztes Natriumhydroxid enthält, weist folgende Zusammensetzung auf (in Klammern : berechnete Analysenwerte für das reine Natriumsalz) :

| | | |
|---|---|---|
| C : | 42,0 % | (43,5 %) |
| H : | 1,5 % | ( 1,2 %) |
| F : | 33,1 % | (34,4 %) |
| N : | 4,4 % | ( 4,2 %) |
| Na : | 8,0 % | ( 6,9 %) |

Wie in Beispiel 13 beschrieben, wird das Grignard-Reagenz aus 17,4 Teilen Magnesiumspänen und 38,4 Teilen 2-Brompyridin in 200 Volumenteilen Tetrahydrofuran hergestellt und mit 21,2 Teilen des oben beschriebenen rohen Natriumsalzes, das in 120 Volumenteilen Diethylether suspendiert ist, umgesetzt. Nach dem Aufarbeiten erhält man 16 Teile rohes 2-Pyridyl-2,8-bis-(trifluormethyl)-4-chinolylketon, das

entspricht 74,3 % der Theorie. Daraus erhält man durch Auskochen mit n-Hexan 13,7 Teile reines Keton, das entspricht 63,6 % der Theorie.

Beispiel 14

2-Pyridyl-2,8-Bis-(trifluormethyl)-4-chinolyl-keton

Man führt die Reaktion wie unter Beispiel 13 beschrieben durch und arbeitet den nach Durchführen der Reaktion und Eindampfen erhaltenen Rückstand wie unter Beispiel 9 beschrieben auf. Man erhält 12,5 Teile reines 2-Pyridyl-2,8-bis-(trifluormethyl)-4-chinolylketon, das sind 70,4 % der Theorie.

Beispiel 15

2-Pyridyl-2,8-bis-(trifluormethyl)-4-chinolyl-keton

Analog Beispiel 1 werden in 100 Volumenteilen Wasser 12,2 Teile Kaliumhydroxid, 43 Teile 7-Trifluormethyl-isatin und 26,8 Teile 1,1,1-Trifluoraceton unter Normaldruck 6 Stunden lang bei ca. 95 °C zur Reaktion gebracht. Die Reaktionslösung wird eingedampft und nach dem Trocknen (36 Stunden bei 130 °C und ca. 20 mbar) erhält man 65 Teile rohes, noch überschüssiges KOH enthaltendes Kaliumsalz der 2,8-Bis-(trifluormethyl)-chinolin-4-carbonsäure, das folgende Zusammensetzung aufweist (in Klammern : berechnete Analysenwerte für das reine Kaliumsalz) :

| | | |
|---|---|---|
| C : | 41,0 % | (41,5 %) |
| H : | 1,4 % | ( 1,2 %) |
| F : | 32,2 % | (32,8 %) |
| K : | 11,0 % | (11,3 %) |
| N : | 4,4 % | ( 4,0 %) |

Analog Beispiel 7 wird das Grignard-Reagenz aus 14,6 Teilen Magnesiumspänen und 32 Teilen 2-Brompyridin in 230 Volumenteilen Tetrahydrofuran hergestellt und mit 22,3 Teilen des oben beschriebenen rohen Kaliumsalzes, das in 100 Volumenteilen Diethylether suspendiert ist, umgesetzt. Nach dem Aufarbeiten (Lösen des Eindampfrückstandes der Grignard-Reaktion in einem Gemisch aus 500 Volumenteilen Wasser und 100 Volumenteilen konz. Salzsäure, Zugabe von 100 Teilen Ethylendiamintetraessigsäure, Einstellen des pH-Wertes mit 50 %iger Natronlauge auf 12, kontinuierliche Extraktion des Gemisches mit 2 000 Volumenteilen Toluol im Perforator und Eindampfen des Toluolextraktes) erhält man 19,5 Teile rohes 2-Pyridyl-2,8-bis-(trifluormethyl)-4-chinolyl-keton, das sind 76,8 % der Theorie. Nach dem Auskochen mit n-Hexan (2 mal mit je 800 Volumenteilen n-Hexan) kann man 14,9 Teile reines Keton isolieren, das sind 58,7 % der Theorie.

Beispiel 16

2-Pyridyl-2,8-bis-(trifluormethyl)-4-chinolyl-keton

In einer Apparatur mit Rührer und Innenthermometer werden unter Argon 2,45 Teile Magnesiumspäne vorgelegt und mit einem Volumenteil der Lösung von 11,5 Teilen 2-Chlorpyridin in 24 Volumenteilen Tetrahydrofuran versetzt. Nach Starten der Grignard-Reaktion durch Zugabe von 0,2 Volumenteilen Ethylbromid gibt man den Rest der 2-Chlorpyridin-Lösung innerhalb von 15 Minuten unter Rühren zu. Dabei erwärmt sich das Reaktionsgemisch auf Siedetemperatur und färbt sich dunkel. Man gibt innerhalb von 50 Minuten weitere 30 Volumenteile Tetrahydrofuran zu und hält das Reaktionsgemisch insgesamt 3 Stunden am Sieden. Das dickflüssige Reaktionsprodukt wird unter Schutzgasatmosphäre vom ungelöst gebliebenen Magnesium abdekantiert und zu 9,5 Teilen Lithiumsalz der 2,8-Bis-(trifluormethyl)-chinolin-4-carbonsäure, die in 100 Volumenteilen Tetrahydrofuran vorgelegt wurde, bei 50 °C unter gutem Rühren zugetropft und noch 2 Stunden bei dieser Temperatur gerührt.

Nach der gleichen Aufarbeitung wie bei Beispiel 15 beschrieben, erhält man 5 Teile rohes 2-Pyridyl-2,8-bis-(trifluormethyl)-4-chinolylketon, das sind 44,8 % der Theorie. Durch Auskochen mit n-Hexan kann man daraus 3 Teile reines Keton erhalten, das sind 26,9 % der Theorie.

Beispiel 17

2-Pyridyl-2,8-bis-(trifluormethyl)-4-chinolyl-keton

31 Teile 2,8-Bis-(trifluormethyl)-chinolin-4-carbonsäure werden in 200 Volumenteilen abs. Ethanol mit 24,9 Teilen Thallium-(I)-ethylat versetzt. Nach dem Eindampfen und Trocknen erhält man 51 Teile des Thallium-(I)-Salzes der 2,8-Bis-(trifluormethyl)-chinolin-4-carbonsäure.

16,4 Teile Magnesium und 32 Teile 2-Brompyridin werden in 200 Volumenteilen Tetrahydrofuran

analog Beispiel 7 zum 2-Pyridylmagnesiumbromid umgesetzt. Die erhaltene, dekantierte Lösung des Grignard-Reagenzes wird unter Argon zur gut gerührten Lösung von 33 Teilen des oben beschriebenen Thalliumsalzes in 100 Volumenteilen Tetrahydrofuran getropft. Dabei wird durch Außenkühlung dafür gesorgt, daß die Innentemperatur 45 °C nicht übersteigt. Nach der unter Beispiel 7 beschriebenen Aufarbeitung erhält man 17 Teile rohes 2-Pyridyl-2,8-bis-(trifluormethyl)-4-chinolyl-keton, das sind 71,3 % der Theorie, daraus durch Auskochen mit n-Hexan 15,2 Teile reines Keton, das sind 63,8 % der Theorie.

Beispiel 18

2-Pyridyl-2,8-bis-(trifluormethyl)-4-chinolyl-keton

Unter Argon wird das Gemisch aus 4,1 Teilen Magnesium, 24 Teilen 2-Brompyridin, 20 Teilen Lithiumsalz der 2,8-Bis-(trifluormethyl)-chinolin-4-carbonsäure, 0,05 Teilen Jod und 1 Volumenteil Tetrachlormethan unter Rühren auf 60 °C erwärmt und nach dem alsbaldigen Einsetzen der Reaktion noch eine Stunde bei 50 °C gerührt. Anschließend wird die Lösung vom Ungelösten dekantiert, mit 5 Volumenteilen Wasser versetzt und eingedampft. Der Eindampfrückstand (64 Teile) wird in 200 Volumenteilen konz. Salzsäure gelöst und die erhaltene Lösung unter Rühren mit 1 500 Volumenteilen Wasser verdünnt. Der dabei gebildete Niederschlag wird abfiltriert, mit Wasser gewaschen und getrocknet. Man erhält 14 Teile reines 2-Pyridyl-2,8-bis-(trifluormethyl)-4-chinolyl-keton, das sind 59,6 % der Theorie.

Beispiel 19

erythro-α-2-Piperidyl-2,8-bis-(trifluormethyl)-4-chinolinmethanol

In einer Hydrierapparatur mit Rührer und Innenthermometer wird das Gemisch aus 1 400 Volumenteilen 95 %igem Ethanol, 7,6 Teilen 37 %iger wäßriger Salzsäure und 25 Teilen eines Hydrierkontaktes, der 5 % Platin auf Aktivkohle enthält (Hersteller Firma Engelhard Inds.), vorgelegt und mit Wasserstoff gesättigt. Anschließend gibt man die Lösung von 25 Teilen 2-Pyridyl-2,8-bis-(trifluormethyl)-4-chinolyl-keton in 200 Volumenteilen 95 %igem Ethanol zu und hydriert bei 800 Rührerumdrehungen pro Minute und 24 °C bei Normaldruck. Nachdem innerhalb von 15 Stunden 6 600 Volumenteile Wasserstoff aufgenommen wurden, entsprechend 4,01 Mol Wasserstoff pro Mol 2-Pyridyl-2,8-bis-(trifluormethyl)-4-pyridyl-keton, bricht man die Hydrierung ab. Durch abfiltrieren der Lösung vom Kontakt und Eindampfen erhält man 26,5 Teile rohes erythro-α-2-Piperidyl-2,8-bis-(trifluormethyl)-4-chinolinmethanol-hydrochlorid, das entspricht 94,6 % der Theorie.

Beispiel 20

2-Trifluormethyl-oximinoacetanilid

In einem Reaktionsgefäß mit Rührer, Rückflußkühler und Innenthermometer wird das Gemisch aus 87 Teilen o-Trifluormethylanilin, 3 000 Teilen Wasser, 600 Teilen Natriumsulfat . 10 $H_2O$, 54 Teilen konzentrierter Salzsäure, 130 Teilen Chloralhydrat und 154 Teilen Hydroxylammoniumchlorid 20 Minuten bei 80 °C gerührt. Beim Abkühlen auf Raumtemperatur scheidet sich zunächst ein Öl ab, das beim fortgesetzten Rühren durchkristallisiert. Die Kristalle werden von der Mutterlauge abgetrennt und getrocknet. Man erhält 100 Teile 2-Trifluormethyl-oximinoacetanilid, das sind 79,8 % der Theorie.

Beispiel 21

7-Trifluormethyl-isatin

Bei 80 °C werden 110 Teile 2-Trifluormethyloximinoacetanilid unter starkem Rühren in 500 Volumenteile konzentrierte Schwefelsäure portionsweise eingetragen. Man hält anschließend noch 30 Minuten bei dieser Temperatur, kühlt dann das Reaktionsgemisch auf Raumtemperatur ab und gießt es auf ca. 4 000 Teile Eis. Der ausgefallene Niederschlag wird abgesaugt, mit Wasser gewaschen und im Wasserstrahlvakuum bei 40 °C getrocknet. Man erhält 80 Teile 7-Trifluormethyl-isatin, das entspricht 78,5 % der Theorie.

**Ansprüche**

1. Verfahren zur Herstellung von erythro-α-2-Piperidyl-2,8-bis-(trifluormethyl)-4-chinolin-methanol aus 2,8-Bis-(trifluormethyl)-chinolin-4-carbonsäure, dadurch gekennzeichnet, daß man 2,8-Bis-(trifluormethyl)-chinolin-4-carbonsäure oder deren Salz mit einem 2-Pyridylmagnesiumhalogenid zum 2-Pyridyl-2,8-bis-(trifluormethyl)-4-chinolyl-keton umsetzt und dieses in an sich bekannter Weise zum erythro-α-2-Piperidyl-2,8-bis-(trifluormethyl)-4-chinolin-methanol hydriert.

2. Verfahren nach Anspruch 1, dadurch gekennzeichnet, daß man das Lithium-, Natrium-, Kalium- oder Magnesium-Salz der 2,8-Bis-(trifluormethyl)-4-chinolin-carbonsäure verwendet.

3. Verfahren nach Anspruch 1, dadurch gekennzeichnet, daß man das 2-Pyridylmagnesiumbromid verwendet.

4. Verfahren nach Anspruch 1, dadurch gekennzeichnet, daß bei der Umsetzung der 2,8-Bis-(trifluormethyl)-4-chinolin-4-carbonsäure mit dem 2-Pyridylmagnesiumhalogenid das Mengenverhältnis des zur Herstellung der Grignard-Verbindung eingesetzten 2-Pyridylhalogenids zur Carbonsäure 4 Mol bis 8 Mol pro Mol und bei Verwendung eines Salzes der 2,8-Bis-(trifluormethyl)-4-chinolincarbonsäure das Mengenverhältnis 2 Mol bis 4 Mol pro Mol beträgt.

5. Verfahren nach Anspruch 1, dadurch gekennzeichnet, daß man die Grignard-Umsetzung in Tetrahydrofuran, Diethylether, Diisopropylether, 1,2-Dimethoxyethan, Diethylenglykoldimethylether oder Methyl-tert.-butyl-ether oder einem Gemisch dieser Ether bei einer Temperatur von 0 bis 80 °C durchführt.

6. Verfahren nach Anspruch 1, dadurch gekennzeichnet, daß man das 2-Pyridylmagnesiumhalogenid in Gegenwart der 2,8-Bis-(trifluormethyl)-chinolin-4-carbonsäure oder eines ihrer Salze herstellt und *in situ* mit der genannten Carbonsäure oder ihren Salzen umsetzt.

7. Verfahren nach Anspruch 1, dadurch gekennzeichnet, daß man das Reaktionsgemisch der Grignard-Umsetzung direkt eindampft, den Eindampfrückstand in wäßriger konzentrierter Salzsäure, wobei pro Teil Eindampfrückstand 1 bis 3 Teile Säure verwendet werden, löst, die Lösung gegebenenfalls filtriert und anschließend das 2-Pyridyl-2,8-bis-(trifluormethyl)-4-chinolylketon durch Verdünnen mit Wasser, wobei pro Teil konzentrierter Salzsäure 1 bis 10 Teile Wasser verwendet werden, ausfällt.

8. Verfahren nach Anspruch 1, dadurch gekennzeichnet, daß man eine 2,8-Bis-(trifluormethyl)-chinolin-4-carbonsäure oder deren Salz verwendet, die bzw. das hergestellt worden ist aus 7-Trifluormethyl-isatin durch Umsetzung mit 1,1,1-Trifluoraceton in Gegenwart einer starken Base.

9. Verfahren nach Anspruch 8, dadurch gekennzeichnet, daß man als starke Base ein Alkalimetallhydroxid verwendet.

10. Verfahren nach Anspruch 8, dadurch gekennzeichnet, daß das molare Mengenverhältnis von 7-Trifluormethyl-isatin zu 1,1,1-Trifluoraceton zu Alkalimetallhydroxid 1 : 1-1,2 : 1-1,2 beträgt.

11. Verfahren nach Anspruch 8, dadurch gekennzeichnet, daß die Umsetzung bei Temperaturen von 60 bis 150 °C unter Normaldruck oder in einem geschlossenen System durchgeführt wird.

12. Verfahren nach Anspruch 8, dadurch gekennzeichnet, daß man ein 7-Trifluormethyl-isatin verwendet, das hergestellt worden ist durch Cyclisierung von 2-Trifluormethyloximinoacetanilid in konzentrierter Schwefelsäure in 10 bis 15 gew.%iger Lösung bei Temperaturen von 75 bis 85 °C.

13. Verfahren nach Anspruch 12, dadurch gekennzeichnet, daß man ein 2-Trifluormethyloximinoacetanilid verwendet, das hergestellt worden ist, aus o-Trifluormethylanilin mit Chloralhydrat und Hydroxylammoniumhydrochlorid in einem molaren Verhältnis von 1 : 1,45 : 4,10 in wäßriger, salzsaurer und natriumsulfathaltiger Lösung mit einem Gehalt von 2,5 bis 3,5 Gew.% o-Trifluormethylanilin, 0,6 bis 0,8 Gew.% Chlorwasserstoff und 17 bis 25 Gew.% Natriumsulfat. 10 $H_2O$ bei Temperaturen von 50 bis 100 °C.

**Claims**

1. A process for the preparation of erythro-α-piperid-2-yl-2,8-bis-(trifluoromethyl)-quinolin-4-yl-methanol from 2,8-bis-(trifluoromethyl)-quinoline-4-carboxylic acid, wherein 2,8-bis-(trifluoromethyl)-quinoline-4-carboxylic acid or a salt thereof is reacted with a pyrid-2-yl-magnesium halide to give pyrid-2-yl 2,8-bis-(trifluoromethyl)-quinolin-4-yl ketone and the latter is hydrogenated in a conventional manner to give erythro-α-piperid-2-yl-2,8-bis-(trifluoromethyl)-quinolin-4-yl-methanol.

2. A process as claimed in claim 1, wherein the lithium, sodium, potassium or magnesium salt of 2,8-bis-(trifluoromethyl)-quinoline-4-carboxyclic acid is used.

3. A process as claimed in claim 1, wherein pyrid-2-yl-magnesium bromide is used.

4. A process as claimed in claim 1, wherein, in the reaction of 2,8-bis-(trifluoromethyl)-quinoline-4-carboxylic acid with the pyrid-2-yl-magnesium halide, the molar ratio of the pyrid-2-yl magnesium halide, the molar ratio of the pyrid-2-yl halide employed in preparing the Grignard compound to the carboxylic acid is 4-8 : 1, whilst if the carboxylic acid is employed in the form of a salt, the corresponding molar ratio is 2-4 : 1.

5. A process as claimed in claim 1, wherein the Grignard reaction is carried out in tetrahydrofuran, diethyl ether, diisopropyl ether, 1,2-dimethoxyethane, diethylene glycol dimethyl ether or methyl tert.-butyl ether or a mixture of these ethers, at from 0 to 80 °C.

6. A process as claimed in claim 1, wherein the pyrid-2-yl-magnesium halide is prepared in the presence of the 2,8-bis-(trifluoromethyl)-quinoline-4-carboxylic acid or of a salt thereof, and is reacted *in situ* with the said carboxylic acid or salt thereof.

7. A process as claimed in claim 1, wherein the mixture from the Grignard reaction is evaporated direct, the evaporation residue is dissolved in aqueous concentrated hydrochloric acid, using from 1 to 3 parts of acid per part of residue, the solution is filtered if necessary, and the pyrid-2-yl-2,8-bis-(trifluoromethyl)-quinolin-4-yl ketone is then precipitated by dilution with water, using from 1 to 10 parts of water per part of concentrated hydrochloric acid.

8. A process as claimed in claim 1, wherein the 2,8-bis-(trifluoromethyl)-quinoline-4-carboxylic acid, or salt thereof, has been prepared from 7-trifluoromethyl-isatin by reaction with 1,1,1-trifluoroacetone in the presence of a strong base.

9. A process as claimed in claim 8, wherein the strong base used is an alkali metal hydroxide.

10. A process as claimed in claim 8, wherein the molar ratio of 7-trifluoromethyl-isatin to 1,1,1-trifluoroacetone to alkali metal hydroxide is 1 : 1-1.2 : 1-1.2.

11. A process as claimed in claim 8, wherein the reaction is carried out at from 60 to 150 °C under atmospheric pressure or in a closed system.

12. A process as claimed in claim 8, wherein the 7-trifluoromethyl-isatin used has been prepared by cyclizing 2-trifluoromethyloximinoacetanilide in 10-15 % strength by weight solution in concentrated sulfuric acid at from 75 to 85 °C.

13. A process as claimed in claim 12, wherein the 2-trifluoromethyloximinoacetanilide used has been prepared from o-trifluoromethylaniline, chloral hydrate and hydroxylammonium chloride in a molar ratio of 1 : 1.45-4.10 by reacting these, at from 50 to 100 °C, in an aqueous hydrochloric acid solution of sodium sulfate, which solution contains from 2.5 to 3.5 % by weight of o-trifluoromethylaniline, from 0.6 to 0.8 % by weight of hydrogen chloride and from 17 to 25 % by weight of sodium sulfate decahydrate.

**Revendications**

1. Procédé de préparation de l'érythro-α-2-pypéridyl-2,8-bis-(trifluorométhyl)-4-quinoléine-méthanol à partir de l'acide 2,8-bis-(trifluorométhyl)-quinoléine-4-carboxylique, caractérisé en ce que l'on transforme l'acide 2,8-bis-(trifluorométhyl)-quinoléine-4-carboxylique ou un sel de cet acide par réaction avec un halogénure de 2-pyridyl-magnésium en 2-pyridyl-2,8-bis-(trifluorométhyl)-4-quinoléyl-cétone et celle-ci par une hydrogénation connue en soi en érythro-α-2-pipéridyl-2,8-bis-(trifluorométhyl)-4-quinoléine-méthanol.

2. Procédé suivant la revendication 1, caractérisé en ce que le sel de l'acide 2,8-bis-(trifluorométhyl)-4-quinoléine-carboxylique est choisi parmi les sels de lithium de sodium, de potassium et de magnésium.

3. Procédé suivant la revendication 1, caractérisé en ce que l'on emploie le bromure de 2-pyridyl-magnésium.

4. Procédé suivant la revendication 1, caractérisé en ce que, pour la réaction de l'acide 2,8-bis-(trifluorométhyl)-4-quinoléine-4-carboxylique et de l'halogénure de 2-pyridyl-magnésium, la proportion de l'halogénure de 2-pyridyle utilisé pour la préparation du dérivé de Grignard est de 4 moles à 8 moles par mole d'acide carboxylique ou de 2 moles à 4 moles par mole, si un sel de l'acide 2,8-bis-(trifluorométhyl)-4-quinoléine-carboxylique est mis en œuvre.

5. Procédé suivant la revendication 1, caractérisé en ce que la réaction de Grignard est réalisée à une température de 0 à 80 °C dans du tétrahydrofuranne, de l'éther diéthylique, de l'éther diisopropylique, du 1,2-diméthoxy-éthane, de l'éther diméthylique de diéthylène-glycol ou de l'éther méthyl-tert.-butylique ou dans un mélange de tels éthers.

6. Procédé suivant la revendication 1, caractérisé en ce que l'halogénure de 2-pyridyl-magnésium est préparé en présence d'acide 2,8-bis-(trifluorométhyl)-quinoléine-4-carboxylique ou d'un de ses sels, puis on le fait réagir *in situ* avec cet acide carboxylique ou le sel de celui-ci.

7. Procédé suivant la revendication 1, caractérisé en ce que le mélange réactionnel de la réaction de Grignard est concentré directement par évaporation, puis le résidu de l'évaporation est repris dans de l'acide chlorhydrique concentré aqueux en utilisant 1 à 3 parties d'acide par partie de résidu, la solution formée est éventuellement filtrée et la 2-pyridyl-2,8-bis-(trifluorométhyl)-4-quinoléyl-cétone est précipitée par addition d'eau à raison de 1 à 10 parties d'eau par partie d'acide chlorhydrique concentré.

8. Procédé suivant la revendication 1, caractérisé en ce que l'on utilise un acide 2,8-bis-(trifluorométhyl)-quinoléine-4-carboxylique, ou un sel de cet acide, préparé par réaction de la 7-trifluorométhyl-isatine en présence d'une base forte avec le 1,1,1-trifluor-acétone.

9. Procédé suivant la revendication 8, caractérisé en ce que la base forte est un hydroxyde d'un métal alcalin.

10. Procédé 1 suivant la revendication 8, caractérisé en ce que les proportions molaires de la 7-trifluorométhyl-isatine, du 1,1,1-trifluor-acétone et de l'hydroxyde de métal alcalin sont dans un rapport de 1 : 1 à 1,2 : 1 à 1,2.

11. Procédé suivant la reveridication 8, caractérisé en ce que la réaction est réalisée à des températures de 60 à 150 °C, à la pression normale ou dans un système fermé.

12. Procédé suivant la revendication 8, caractérisé en ce que l'on emploie une 7-trifluorométhyl-isatine préparée par cyclisation du 2-trifluorométhyl-oximino-acétanilide en solution de 10 à 15 % en poids dans de l'acide sulfurique concentré à des températures de 75 à 85 °C.

13. Procédé suivant la revendication 12, caractérisé en ce que l'on emploie un 2-trifluorométhyl-oximino-acétanilide préparé à partir de o-trifluorométhyl-aniline, d'hydrate de chloral et de chlorhydrate d'hydroxyl-ammonium dans un rapport molaire de 1 : 1,45 : 4,10 dans une solution aqueuse contenant de

l'acide chlorhydrique et du sulfate de sodium avec des concentrations de 2,5 à 3,5 % en poids de o-trifluorométhyl-aniline, de 0,6 à 0,8 % en poids d'hydrogène chloré et de 17 à 25 % en poids de sulfate de sodium décahydraté, à des températures de 50 à 100 °C.